(19) Europäisches Patentamt
European Patent Office
Office européen des brevets

(11) Publication number : **0 463 707 A1**

# EUROPEAN PATENT APPLICATION

(21) Application number : **91202030.2**

(22) Date of filing : **17.12.86**

(51) Int. Cl.⁵ : **C12N 15/52,** C12N 15/76, C12P 17/02, C12Q 1/68, **C12N 1/20**

This application was filed on 07.08.1991 as a divisional application to the application mentioned under INID code 60.

(30) Priority : **17.12.85 GB 8531036**
**10.07.86 GB 8616851**

(43) Date of publication of application :
**02.01.92 Bulletin 92/01**

(84) Designated Contracting States :
**AT BE CH DE FR GB IT LI LU NL SE**

(60) Publication number of the earlier application in accordance with Art. 76 EPC : **0 262 154**

(71) Applicant : **LUBRIZOL GENETICS INC.**
**29400 Lakeland Boulevard**
**Wickliffe Ohio 44092 (US)**

(72) Inventor : **Humphreys, Gwynfor Owen**
**20 Tattersall Close**
**Wokingham, Berkshire RG11 2LP (GB)**
Inventor : **Bailey, Christopher Richard**
**1 Flitchfold Villas**
**Loxwood, West Sussex RH14 0RQ (GB)**
Inventor : **McKillop, Christine Ann**
**5 Denham Close**
**Maidenhead, Berkshire (GB)**

(74) Representative : **Fisher, Adrian John et al**
**CARPMAELS & RANSFORD 43 Bloomsbury Square**
**London WC1A 2RA (GB)**

(54) **Production of polyketide antibiotics.**

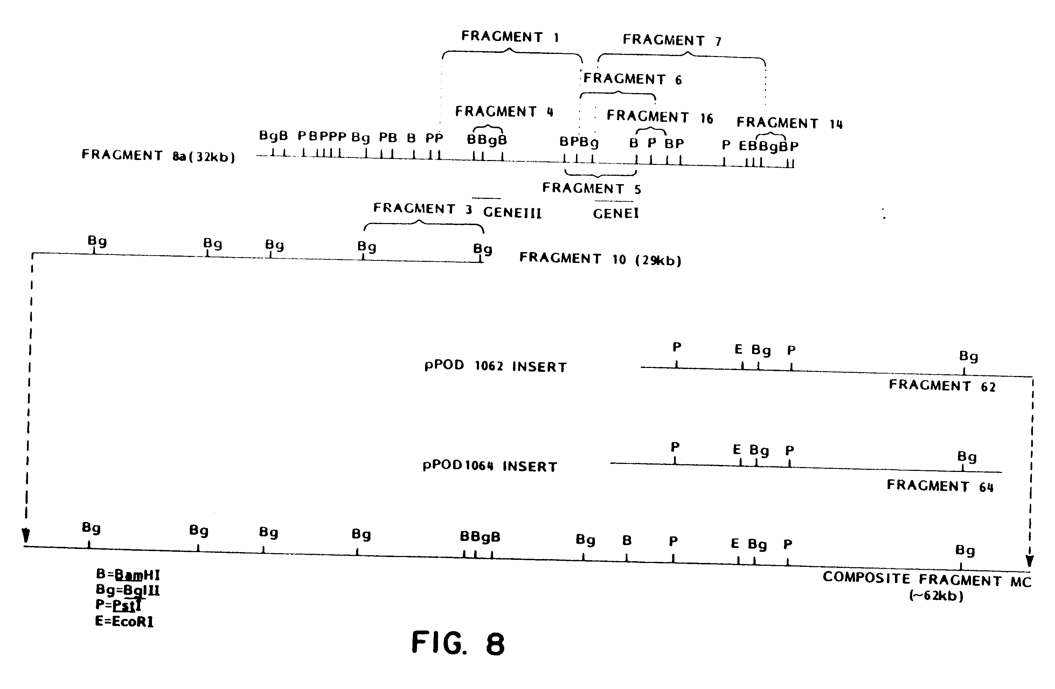

(57) A method for producing a milbemycin in a naturally non-milbemycin producing strain of a bacterium of the genus Streptomyces comprises introducing into the non-producing strain the DNA composite fragment MC (Figure 8) or a functionally equivalent DNA fragment.

EP 0 463 707 A1

FIG. 8

Field of the Invention

The present invention relates to DNA sequences containing genes for the biosynthesis of milbemycin, and to the use of such DNA sequences in the production of polyketide antibiotics.

Background of the Invention

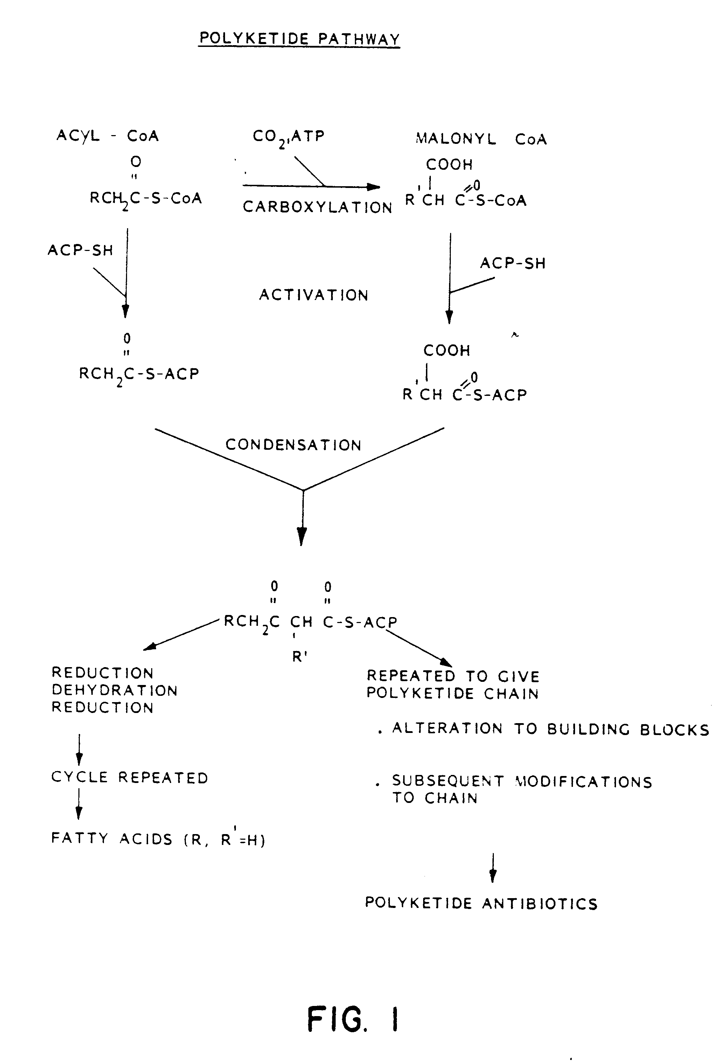

Polyketides are a structurally and functionally diverse group of natural products which are related by similarities in their biosynthesis rather than by particular structural similarities. Polyketide natural products have structures that are in a broad sense derived from a similar basic structure which consists of poly-$\beta$-ketomethylene chains ($-[CHRC0]_n-$, where n can range from about 4 to 20). Polyketides are distinguished from other natural products by the mechanism by which this basic structure is synthesized. A schematic diagram for the polyketide pathway is presented in Figure 1. The poly-$\beta$-ketomethylene chain is synthesized from a "starter" unit, an acyl-CoA ester, by stepwise condensations of malonyl-CoA units with concomitant decarboxylation steps. The acyl-CoA ester "starter" unit varies depending on the particular polyketide. The added malonyl units also vary with the polyketide and can be methyl malonyl, ethyl malonyl, and other alkyl malonyl groups.

It is believed that in polyketide biosynthesis the acyl and malonyl units are assembled by a multi-enzyme complex, generically called a polyketide synthase which accepts the precursor units, effects condensation and other ancillary reactions with intermediates remaining enzyme-bound until the ultimate stabilized polyketide is formed and released. Only a very few polyketide synthases have been characterized; however, polyketide synthases are believed to be similar in that they incorporate a site for binding of the acyl-CoA "starter" molecule, and have transacylase functions which effect condensations. Polyketide synthases, while related, are specific to the particular polyketide that is synthesized. Polyketide synthases can display considerable differences in their specificity for different acyl-CoA "starter" molecules, specificity for different malonyl-CoA units, specificity for the particular order in which malonyl units are added to the chain, and for the presence of ancillary reactions occurring in association with the construction of the basic polyketide chain, including, among others, reductions, substitutions and cyclizations.

Such differences likely reflect functional as well as structural differences in the enzymes (and in the genes encoding them.) involved in the construction of the basic polyketide structure of individual polyketides. Thus, while there are some mechanistic features of polyketide biosynthesis that are common, there is no evidence that the particular enzymes involved are structurally related.

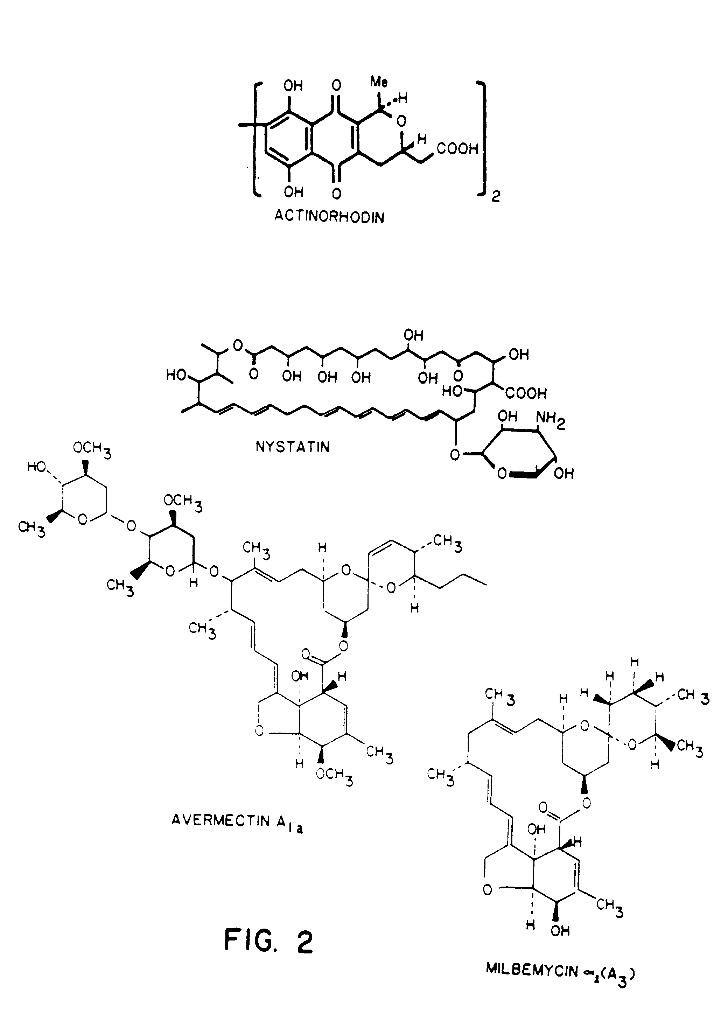

Several families of antibiotics produced by members of the actinomycetes, particularly bacteria of the genus Streptomyces, belong to the polyketide group of natural products. Polyketide antibiotics include the tetracyclines (e.g. oxytetracycline), anthracyclines (e.g. daunorubicin), macrolides (e.g. erythromycin), polyenes (e.g. amphotericin B), polyethers (e.g. monensin), ansamycins (e.g. rifamycin), isochromanequinones (e.g. actinorhodin), avermectins and milbemycins ("Economic Microbiology", Vol. 3, "Secondary Products of Metabolism", Ed. Rose, A.H., 1979, Academic Press, pages 355-382; Cane, D.E. and Liang, T-C., (1983) J. Am. Chem. Soc. 105:4110-4112; and Hopwood, D.A., Malpartida, F., Keiser, H.M., Ikeda, H., Duncan, J., Fujii, I., Rudd, B.A.M., Floss, F.G. and Omura, S. (1985) Nature 314:642-644). As seen in Figure 2, which provides structures of several polyketide antibiotics from different families, polyketide antibiotics are structurally diverse. The polyketide antibiotics represent a functionally diverse group including bactericides, fungicides, nematicides, insecticides and cytotoxic agents. It is believed that these antibiotics are synthesized via a polyketide pathway (vide supra), Figure 1).

Summary of the Invention

It is an object of this invention to provide a method for producing a milbemycin antibiotic in a strain of a bacterium of the genus Streptomyces which does not naturally produce the antibiotic. This method involves the introduction of DNA fragments containing isolated genes or gene clusters involved in the biosynthesis of milbemycin into a suitable non-producing Streptomyces host strain wherein the introduced biosynthetic genes are expressed and the desired milbemycin is produced.

It is also an object of this invention to provide DNA fragments containing isolated milbemycin biosynthetic genes and gene clusters, DNA vectors containing these fragments and genes as well as bacterial strains which contain these vectors and DNA fragments.

In one aspect, the present invention provides a method for the production of milbemycins, by introduction of DNA composite fragment MC (Figure 8) or a DNA fragment that is functionally equivalent to fragment MC into a milbemycin non-producing strain of Streptomyces. Bacterial hosts which are suitable for use in this method are strains of bacteria of the genus Streptomyces which do not naturally produce the desired milbemycin. Suitable host strains are those that synthesize the necessary biochemical precursors for synthesis of the antibiotic, e.g. the appropriate acyl-CoA and malonyl-CoA precursors, and that are transformable. It is contemplated that Streptomyces lividans is a suitable host strain.

The present invention provides, in another aspect, a method for activating the expression of a polyketide antibiotic biosynthetic gene cluster in a bacterial strain of the genus Streptomyces which strain contains the biosynthetic genes sufficient for the production of the polyketide antibiotic but lacks a functional polyketide antibiotic biosynthesis activator gene which comprises introducing into said bacterial strain a DNA fragment which comprises milbemycin Gene II.

In particular it is contemplated, in a specific embodiment, that this method for activating expression of synthesis of a polyketide antibiotic can be applied to synthesis of actinorhodin, milbemycin and avermectin and particularly to the biosynthesis of one of these antibiotics in strains of Streptomyces lividans, Streptomyces coelicolor and Streptomyces avermitilis.

It is also contemplated that this method may also be employed to enhance the level of production of a polyketide antibiotic by introduction of milbemycin Gene II into a strain which naturally produces the polyketide antibiotic.

The present invention provides, in yet another aspect, DNA fragments and vectors containing these fragments which are useful in the methods of the present invention. In particular, vectors are provided which comprise DNA fragments which consist essentially of the milbemycin biosynthetic gene cluster, preferably where the DNA fragment is fragment MC. Vectors which comprise DNA molecules consisting essentially of the DNA sequence encoding a milbemycin biosynthetic gene are also provided, including those encoding milbemycin Gene I, milbemycin Gene II and milbemycin Gene III. Vectors which comprise DNA molecules that are DNA fragment 1, fragment 2, fragment 3, fragment 4, fragment 5, fragment 6, fragment 7, fragment 8a, fragment 8b, fragment 10, fragment 12, fragment 14, fragment 16, fragment 62 and fragment 64 are also provided. The vectors and DNA fragments provided herein are useful as hybridization probes or in some cases for introduction into appropriate host bacteria in order to effect synthesis of polyketide antibiotics or to effect a modification of a polyketide antibiotic naturally produced by the host strain.

With respect to the hybridization probes provided in the present invention it is contemplated that in addition to the specific fragments identified, fragments that are functionally equivalent as hybridization probes to the specific DNA fragments identified are also part of the present invention. This includes hybridization probes that consist essentially of the sequences of the fragments identified herein, as well as hybridization probes whose sequences are derived from the sequences of the fragments identified herein.

## Brief Description of the Figures

Figure 1 is a schematic diagram of the polyketide pathway.

Figure 2 shows the structural formulae of four representative polyketide antibiotics;

Figure 3 provides restriction enzyme maps of the S. coelicolor DNA fragments inserted into plasmids pIJ2303, pIJ2305 and pIJ2308; the location of certain actinorhodin genes within these inserts is also provided (Malpartida and Hopwood, (1986) Mol. Gen. Genet. 205:66-73).

Figure 4 shows the results of Southern hybridization experiments using the pIJ2305 fragment as a probe of PstI digests of total chromosomal DNA of (1) S. coelicolor, (2) S. lividans and (3) Streptomyces sp. B41-146.

Figure 5 shows the restriction map of vector pIJ610;

Figure 6 shows the restriction map and relative arrangement of the fragments 8a, 8b and 10. The location of fragments, 1, 2, 3, 4, 5, 6, 7, 14 and 16 is also provided.

Figure 7 shows the scheme for transferring the clone 8a insert into vector pIJ 922.

Figure 8 shows the restriction map and relative arrangement of the fragments 8a, 62 and 64.

Figure 9 shows a restriction map of the phage vector pP0D9.

Figure 10 shows the theoretical insertion of pP0D11 into the Streptomyces sp. B41-146 chromosome compared to the structure of one actual insertion event.

Figure 11 shows the theoretical insertion of pP0D12 into the Streptomyces sp. B41-146 chromosome compared to the structure of one actual insertion event.

Figure 12 shows the theoretical insertion of pP0D1071 into the Streptomyces sp, B41-146 chromosome compared to the structure of one actual insertion event.

Figure 13 shows the theoretical insertion of pP0D1072 into the Streptomyces sp. B41-146 chromosome compared to the structure of one actual insertion event.

Detailed Description of the Invention

The group of polyketide natural products are defined by an overall similarly in the biosynthetic pathway by which they are synthesized in vivo and similar precursors may be involved in the synthesis of the basic polyketide chain. There is, however, much structural diversity among polyketides in general and specifically among the various polyketide antibiotics. The structural diversity of the polyketide antibiotics reflects differences in their biosynthetic pathways. It is believed that major structural variations of the stabilized polyketide core are mediated not only by subsequent enzymatic modification of the basic polyketide chains but in most cases by a variety of enzymatic activities that are directly associated with the synthesis of the basic polyketide chain.

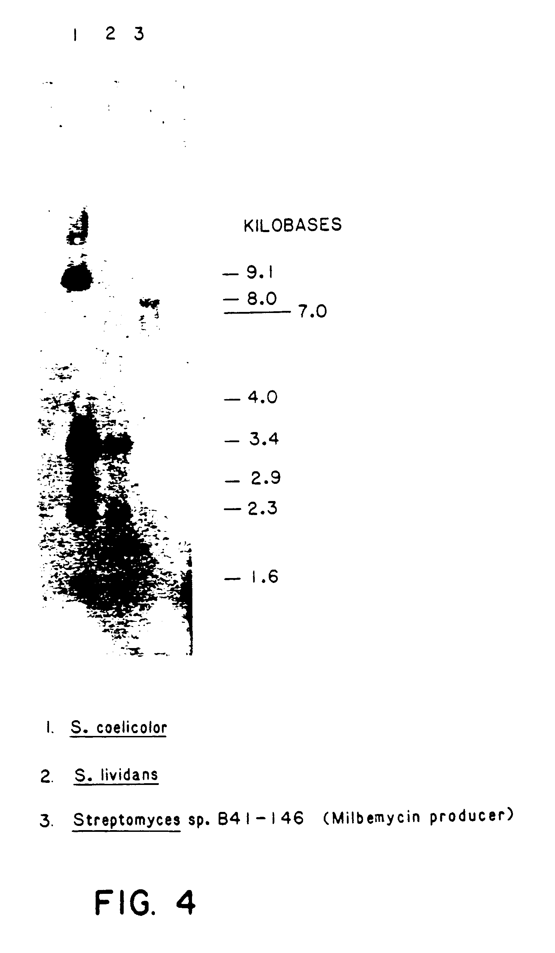

In the present work it is demonstrated that DNA fragments containing actinorhodin biosynthetic genes specifically hybridize to DNA fragments of a strain of Streptomyces, Streptomyces sp. B41-146, which produces the nematocidal polyketide antibiotic milbemycin (Figure 4). DNA hybridization is demonstrated between the actinorhodin biosynthetic genes, Gene I and Gene III, and Streptomyces sp. B41-146 DNA fragments that have been shown to contain genes involved in the biosynthesis of milbemycin.

Actinorhodin Gene I and Gene III mutants are found to be blocked at the earliest steps in the biosynthesis pathway of actinorhodin (Malpartida and Hopwood, 1986). It is believed that these genes may be associated with the actinorhodin polyketide synthase. By analogy to the actinorhodin genes, the specific Streptomyces sp. B41-146 DNA that hybridizes to act Gene I is designated milbemycin Gene I and that which hybridizes to act Gene III is designated milbemycin Gene III. Again by analogy to these actinorhodin genes, it is believed that milbemycin Gene I and milbemycin Gene III are associated with early steps in the biosynthesis of milbemycin, possibly associated with milbemycin polyketide synthase.

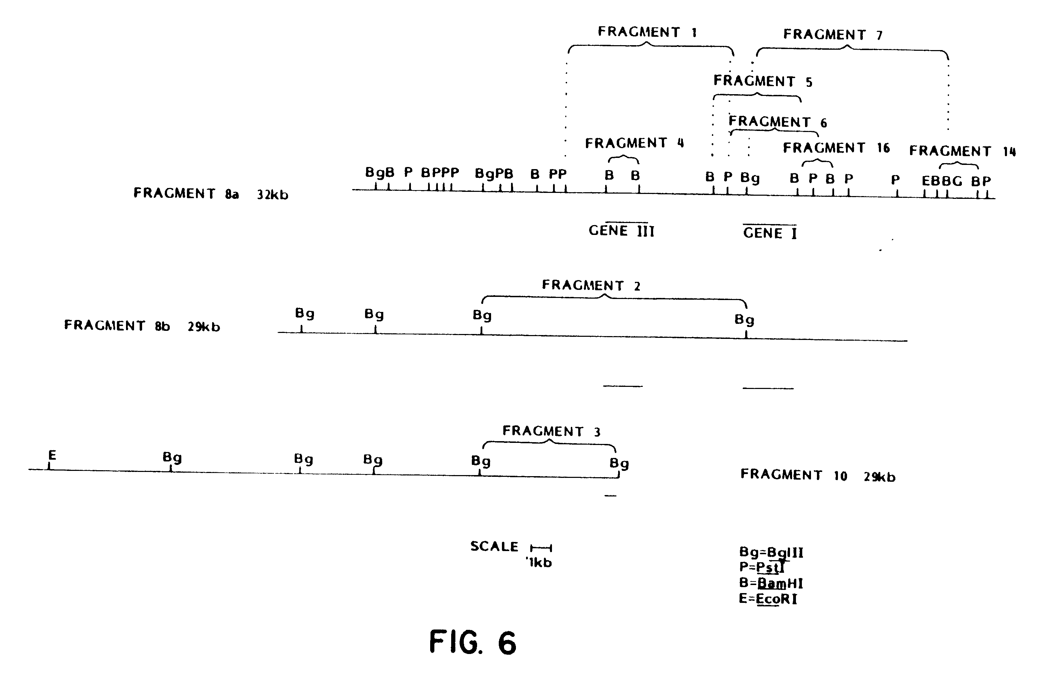

The actinorhodin genes were in fact employed as probes in hybridization screens of a library of Streptomyces sp. B41-146 cloned DNA. Hybridization screens using an act Gene III probe results in the isolation of several DNA fragments (fragments 8a, 8b and 10, Figure 6). It was demonstrated by restriction enzyme mapping that these B41-146 DNA fragments overlap one another and span an approximately 48 kb long contiguous region. Act Gene III probe hybridization within this region was localized to a 1.6. kb BamHI restriction fragment, numbered fragment 4. The act Gene III probe also hybridized to fragments 1, 2 and 3 which all include fragment 4 sequence. Using similar techniques, act Gene I probe hybridization was localized within the cloned B41-146 DNA to fragments 8a, 8b, 5, 6 and 7. Fragment 10 did not hybridize to act Gene I sequences. The regions that hybridize to both act probes are contained on fragment 8a and 8b.

Table 1 presents the results of complementation experiments, in which B41-146 fragments containing sequences that hybridized to act Gene I and act Gene III sequences were introduced into Streptomyces coelicolor mutants blocked in act Gene I (TK 17) or act Gene III (TK 18). As seen in Table 1, the B41-146 fragments contain DNA sequences which appear to complement functionally both act Gene I and act Gene III and the complementary sequences correspond to those regions showing hybridization to the actinorhodin gene probes. The interpretation of the results of Table 1 are complicated, however, by the leaky phenotype of the TK 17 mutant.

As part of the characterization of the B41-146 fragments isolated in hybridization experiments, subfragments of the isolated DNA were introduced into Streptomyces lividans. This Streptomyces species contains the genes for the production of actinorhodin but does not normally produce this polyketide antibiotic. The presence of actinorhodin genes in S. lividans DNA has been confirmed by the presence within S. lividans populations of a small number (usually less than 1%) of individuals which produce actinorhodin. These individuals are likely to be spontaneous mutants.

It was found that when B41-146 fragment 7 was inserted in either orientation or when fragment 12 was inserted in one orientation all transformed S. lividans produced actinorhodin. The B41-146 DNA fragments therefore contain sequences that activate synthesis of the polyketide antibiotic actinorhodin. It has also been demonstrated that fragment 7 contains sequences, a gene, that functionally complements a Streptomyces coelicolor act Gene II mutation. The act Gene II is believed to be involved in the regulation of actinorhodin biosynthesis. The B41-146 DNA sequence that functionally complements act Gene II is designated milbemycin Gene II and is by analogy expected to be involved in the regulation of milbemycin biosynthesis. Fragment 7 does not show hybridization to act Gene II sequences, and is therefore not structurally similar to the actinorhodin regulatory gene. Act Gene II also activates actinorhodin production in transformed S. lividans. Horinouchi and Beppu (1984) Agric. Biol. Chem. 48:2131-2133 have reported the isolation of a regulatory gene, afsB, which controls biosynthesis of A-factor and which also controls the production of the pigments, actinorhodin and prodigiosin. There is at present no indication that afsB is equivalent to either act Gene II or milbemycin Gene II.

It may be the case that many strains of Streptomyces contain silent biosynthetic genes for secondary metabolite production, including polyketide antibiotic production. The milbemycin Gene II sequence and fragments containing it are useful in the activation of such silent polyketide antibiotic biosynthetic genes in strains like S. lividans and may be widely applicable in other Streptomyces species. The milbemycin Gene II sequence may also be useful in the enhancement of the level of production of a polyketide antibiotic in a Streptomyces strain that naturally produces the antibiotic.

The presence of milbemycin Gene II sequence at the far right hand end of fragment 8a, suggests that other genes associated with the biosynthesis of milbemycin could be found in the B41-146 chromosome in the region adjacent to the right of the region spanned by fragment 8a. In order to isolate and clone this adjacent chromosomal DNA, a subfragment (fragment 14) at the far right end of 8a was selected for use as a hybridization probe for a second clone library B41-146 DNA. Two clones were selected in such hybridization screens which contained inserts which extended in the rightward direction from the end of fragment 8a. These fragments are called 62 and 64 and are compared to fragment 8a and 10 in Figure 8. Similar techniques can be employed to isolate DNA sequences which extend in the leftward direction from a desired fragment. Application of such techniques has been termed chromosome "walking".

Fragments 10, 8b, 8a, 62 and 64 define and span a chromosomal region of about 62 kb in length. It is believed that this region contains the complete biosynthetic gene cluster for the production of milbemycin. A composite fragment, fragment MC can be constructed by appropriate restriction enzyme cleavages, ligations and isolation of any intermediate DNA fragments and the desired MC fragment by conventional DNA isolation methods. Fragment MC spans 62 kb of DNA; however, by analogy with other polyketide antibiotics for which the complete biosynthetic gene cluster has been cloned, the milbemycin gene cluster should range in size between about 30 to 40 kb of DNA. It is therefore likely that fragment MC contains in addition to the milbemycin biosynthetic gene cluster, other genes not involved in the biosynthesis. It is likely, based on the mapped locations of the milbemycin Gene I, II and III regions that DNA regions that are not essential for milbemycin production are located at the leftward end of fragment MC. Delineation of the region essential for milbemycin biosynthesis can be done by iterative removal of sequences at the left or right end of fragment MC coupled with intermediate testing of the resultant shorter DNA fragment for the ability to direct synthesis of milbemycin in a bacterial host which does not naturally produce milbemycin (e.g. S. lividans). Fragment MC and fragments derived therefrom that are essentially equivalent in function to fragment MC, in that they contain the complete biosynthetic gene cluster for milbemycin production, are useful in methods described herein for the production of milbemycin in non-milbemycin producing strains of Streptomyces.

An attempt was made to further characterize the milbemycin biosynthetic gene cluster by use of insertional inactivation techniques which had been previously described by Chater and Bruton (1983) Gene 26:67-78. However, the results of these experiments were unexpectedly different from those described by Chater and Bruton. In this method, cloned DNA fragments are inserted into a defective ⌀C31 vector in which the attachment site has been deleted. Phage insertion into the chromosome of an infected strain results through homologous recombination mediated by the inserted cloned DNA. Recombination occurs at the chromosomal sequences which hybridize to the cloned DNA. Theoretically, phage insertion will only cause the disruption of gene expression when the cloned DNA is internal to a transcription unit (i.e. contains neither the 5′ nor the 3′ end sequences of the transcription unit). It was found, however, using the DNA fragments and strains described herein that phage insertion most often induced disruption of gene expression. This was observed even with cloned DNA fragments, like fragment 4, which were believed to contain entire transcription units. It was found that this technique induced the deletion of both phage as well as chromosomal DNA. The technique therefore proved to be a method for the production of milbemycin non-producing mutants. The results of these experiments coincidentally provided another demonstration that the cloned B41-146 DNA of fragment MC contained genes associated with milbemycin production, since deletion of chromosomal DNA in this region resulted in non-producing mutants.

Fragments of B41-146 DNA that contain milbemycin Gene I and milbemycin Gene III, particularly fragments 4 and 5, are expected to be useful, as were the act Gene I and act Gene III probes, as hybridization probes in the isolation of biosynthetic genes of a variety of polyketide antibiotics. These fragments and also other B41-146 DNA fragments in the vicinity of the Gene I and Gene III sequences are expected to be particularly useful in the isolation of biosynthetic genes for polyketide antibiotics which are structurally similar to milbemycin, for example, avermectins.

It has recently been reported (Malpartida et al. (1987) Nature, 325:818-827) that the act Gene I and act Gene III sequences show specific hybridization to DNA from a number of polyketide antibiotic producing strains of Streptomyces, including strains which produce the anthracyclines: tetracenomycin and adriamycin; the macrolides: spiramycin, tylosin, pikromycin and oleandomycin; the polyethers: nonactin, salinomycin, lasalocid and monensin; as well as other isochromanequinone antibiotics. This report further supports the contention that the act Gene I, act Gene III, milb Gene I and milb Gene III probes will be generally useful for the isolation of biosynthetic genes of a variety of polyketide antibiotics.

The methods of the invention involve the use of recombinant DNA techniques which are known per se and therefore do not form part of the invention. For instance, the preparation of clone libraries and the technique of hybridization screening are well known to a person skilled in the art. Thus, a skilled person will be able to carry out the methods of the present inventions using only his ordinary skill and knowledge of the art. A number of techniques that are standard in the art are described in: Maniatis et al. (1982) Molecular Cloning, Cold Spring Harbor Laboratory, Cold Spring Harbor, New York; Wu (ed.) (1979) Meth. Enzymol. 68; Wu et al. (eds.) (1983) Meth. Enzymol. 100 and 101; Grossman and Moldave (eds.) Meth. Enzymol. 65; Miller (ed.) (1972) Experiments in Molecular Genetics, Cold Spring Harbor Laboratory, Cold Spring Harbor, New York; Old and Primrose (1981) Principles of Gene Manipulation, University of California Press, Berkeley; Schleif and Wensink (1982) Practical Methods of Molecular Biology; Glover (ed.) (1985) DNA Cloning Vol. I and II, IRL Press, Oxford, U.K.; Hames and Higgins (eds.) (1985) Nucleic Acid Hybridization, IRL Press, Oxford, U.K.

## Example 1: Hybridization using actinorhodin biosynthetic gene probes

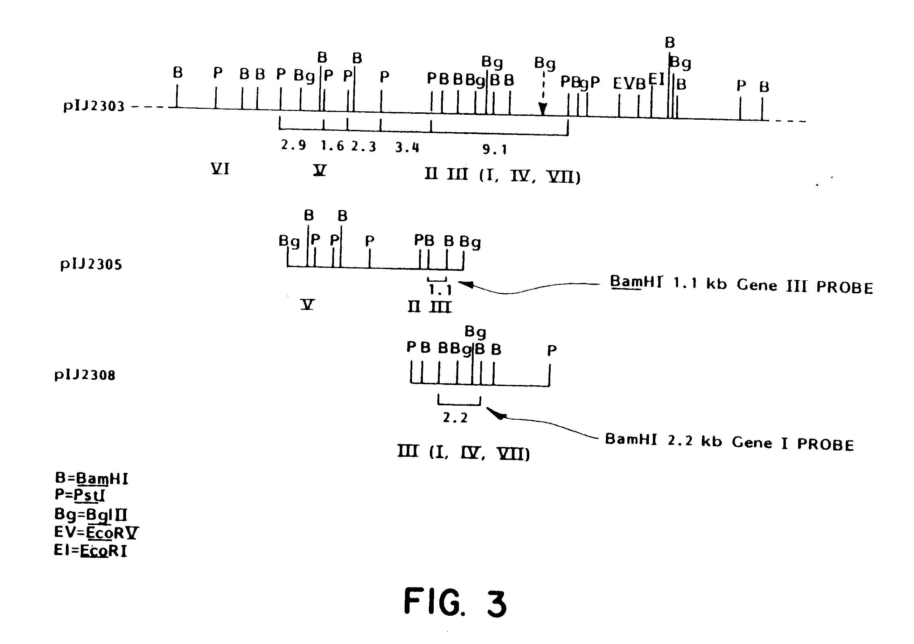

Malpartida and Hopwood (1984) Nature 309:462-464 have reported a recombinant plasmid pIJ2303 which contains a fragment of S. coelicolor DNA approximately 33 kb in length which contains the genes involved in biosynthesis of the polyketide antibiotic actinorhodin. A restriction enzyme map of the insert of pIJ2303 is shown in Figure 3, which also includes the locations of numbered actinorhodin genes within the DNA insert (Malpartida and Hopwood (1986) Mol. Gen. Genet. 205:66-73). Two plasmids, wich contain subfragments of the pIJ2303 insert, designated pIJ2305 and pIJ2308 were obtained from BglII and PstI digests, respectively, of pIJ2303. Figure 3 also provides the restriction maps of the pIJ2305 and pIJ2308 inserts as well as the physical location of actinorhodin genes within these inserts.

PstI digests of DNA derived from S. coelicolor, S. lividans and S. hygroscopicus subsp. aureolacrimosus were probed using the Southern hybridization technique with radioactively labelled insert of pIJ2305. S. coelicolor and S. lividans contain the genes for actinorhodin biosynthesis. S. hygroscopicus subsp. aureolacrimosus, which is commonly known as Streptomyces sp. B41-146 (available as NRRL No. 5739), produces the polyketide antibiotic milbemycin (see Figure 2 for structure). Southern hybridization was performed by standard techniques (Maniatis et al. (1982) Molecular CloningCold Spring Harbor Laboratory; Hames and Higgins (eds) (1985) Nucleic Acid Hybridization IRL Press Oxford). More specifically, filters were prehybridized for 2 hrs at 37°C in a solution containing 6x SSC (Salt-Sodium Citrate, where 1xSSC is 0.15M NaCl, 0.15M trisodium citrate, pH 7.0)/ 50% v/v formamide; 0.1% wt/v SDS (Sodium dodecyl sulfate) and 50 μg/ml salmon sperm DNA. Hybridization was performed at 37°C, overnight using the same solution. Filters were washed twice. First at 37°C for 1 hr. with a 6xSSC/50% v/v formamide/0.1% wt/v SDS solution followed by a second wash at 37°C for 40 min. with 6xSSC.

Southern hybridization results are shown in Figure 4. The pIJ2305 insert probe hybridized to S. coelicolor DNA PstI fragments of 9.1, 3.4, 2.9, 2.3 and 1.6 kb, as expected, A similar hybridization pattern was observed with S. lividans DNA fragments. The actinorhodin gene probe insert of pIJ2305 also hybridized to several fragments of Streptomyces sp. B41-146 DNA, having sizes of approximately 8, 7 and 4 kb.

In order to identify the region of insert pIJ2305 which hybridized to Streptomyces sp. B41-146 DNA fragments, BamHI fragments of the pIJ2305 insert were subcloned, labelled and used as probes in hybridization experiments with Streptomyces sp. B41-146 DNA digests. In these Southern hybridizations, the prehybridization and hybridization steps were performed as above. However, filters were washed at 37°C for 1 hr using 6xSSC/ 50% v/v formamide/ 0.1% wt/v SDS followed by two subsequent 1 hr washes at 37°C using 2x SSC/ 50% v/v formamide. These hybridization experiments showed conclusively that the 1.1$^{kb}$ BamHI fragment of the pIJ2305 insert which contains the entire transcription unit for actinorhodin Gene III (Figure 3) hybridizes specifically to an approximately 8.0 kb fragment of Streptomyces sp. B41-146 DNA.

A second actinorhodin gene probe specific for Gene I was prepared by digesting the pIJ2308 insert with BamHI and isolation of a 2.2 kb fragment which contains actinorhodin Gene I.

## Example 2: Hybridization screening of a Streptomyces sp. B41-146 clone library with actinorhodin gene probes

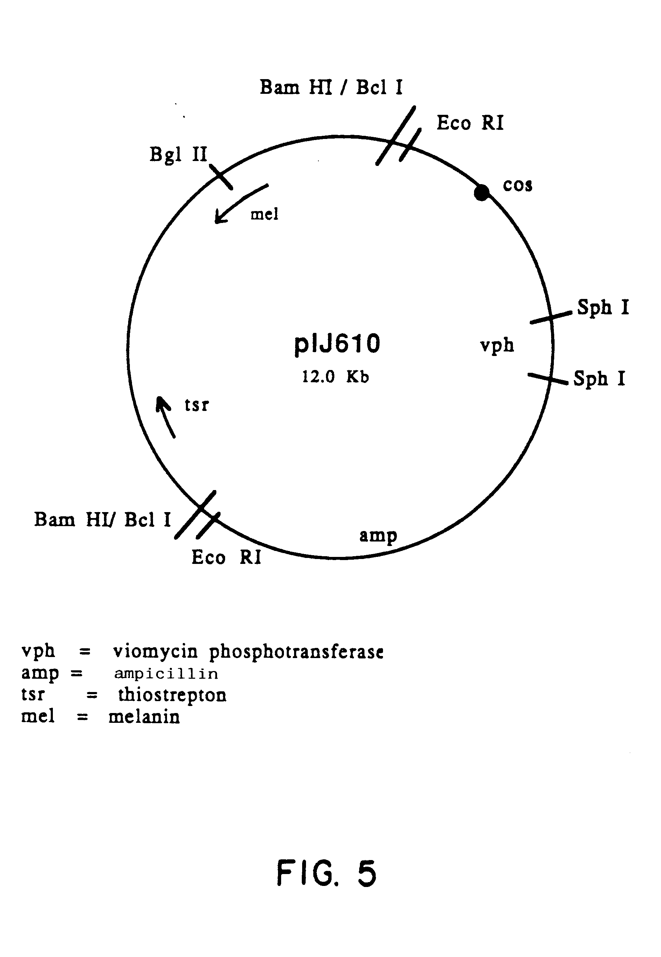

A cosmid library of Streptomyces sp. B41-146 DNA was prepared by Sau3a digestion to give 20-30 kb fragments. The fragments were cloned into the BglII site of the snuttle cosmid pIJ610 , which can be obtained from Tobias kleser, john Innes Institute, using in vitro packaging techniques in Escherichia coli. A restriction map of vector pIJ610 is shown in Figure 5. Selection for inserts is made on the basis of in vitro packaging size constraints in conjunction with phosphatased vector.

The cosmid library thus prepared was screened by hybridization to the 1.1 kb BamHI fragment containing actinorhodin Gene III (vide supra). Conventional techniques were used. Specifically, filters were prehybridized at 37°C for 2.5 hr using a solution of 6xSSC/ 50% v/v formamide/ 0.1% SDS/ 50 μg/ml salmon sperm DNA. Hybridization was carried out overnight at 37°C using the same solution. Filters were washed at 37°C for 70 min. using 6xSSC/ 50% v/v formamide. Of 1,000 clones screened, 3 were found to hybridize to the actinorhodin Gene III probe. These clones were designated 8a, 8b, and 10. The fragments of Streptomyces sp. B41-146 DNA contained in these clones were also designated 8a, 8b and 10, Restriction maps of these fragments are shown in Figure 6. The approximate sizes of fragments 8a, 8b and 10 are 32 kb, 29 kb and 29 kb, respectively, Using conventional restriction enzyme analysis it was shown that these fragments overlap one another and span a contiguous region of Streptomyces sp, B41-146 DNA of about 48 kb.

Restriction subfragments of 8a, 8b and 10 were screened for hybridization to the 1.1kb BamHI actinorhodin Gene III specific probe. Southern filters were prehybridized for 2 hrs as in Example 1 and hybridization was carried out overnight, again as in Example 1. Filters were washed twice at 37°C. The first wash was for 1 hr using 6x SSC/ 50% v/v formamide, the second wash was for 1 hr using 2x SSC/ 50% v/v formamide. The following fragments were found to hybridize to the Gene III specific probe:

An 8 kb PstI subfragment of both 8a and 8b, designated fragment 1, approximately 4 kb of which is also present in 10;

A 13 kb BglII subfragment of 8a and 8b, designated fragment 2;

A 7 kb BglII subfragment of 10, designated fragment 3; and

A 1.6 kb BamHI subfragment of 8a and 8b, designated fragment 4, approximatety 1 kb of which is also present in 10. The location of fragments 1-4 is provided in Figure 6.

The fragments 8a, 8b and 10 and restriction subfragments thereof were also analyzed for hybridization to the 2.2 kb BamHI actinorhodin Gene I specific probe (vide supra). Again conventional Southern hybridization techniques were employed. Specifically, filters were prehybridized for 2 hr and hybridization was conducted at 37°C overnight as described above. Filters were washed at 37°C three times: first for 1 hr with 6x SSC/ 50% v/v formamide; then for 90 min with 2x SSC/ 50 % v/v formamide and then for 90 min with 1x SSC/ 50% v/v formamide. The Gene I specific probe was found to hybridize to both fragments 8a and 8b, but not to fragment 10. Within fragments 8a and 8b, the probe was found to hybridize to a 4.2 kb BamHI subfragment, designated fragment 5; a 4.2kb PstI subfragment, designated fragment 6; and a 10 kb BglII subfragment, designated fragment 7 (see figure 6).

It can be seen in Figure 6 that fragment 8a includes sequences that hybridize to both actinorhodin gene specific probes.

## Example 3: Complementation of S. coelicolor actinorhodin(-) mutants with Streptomyces sp. B41.146 DNA fragments

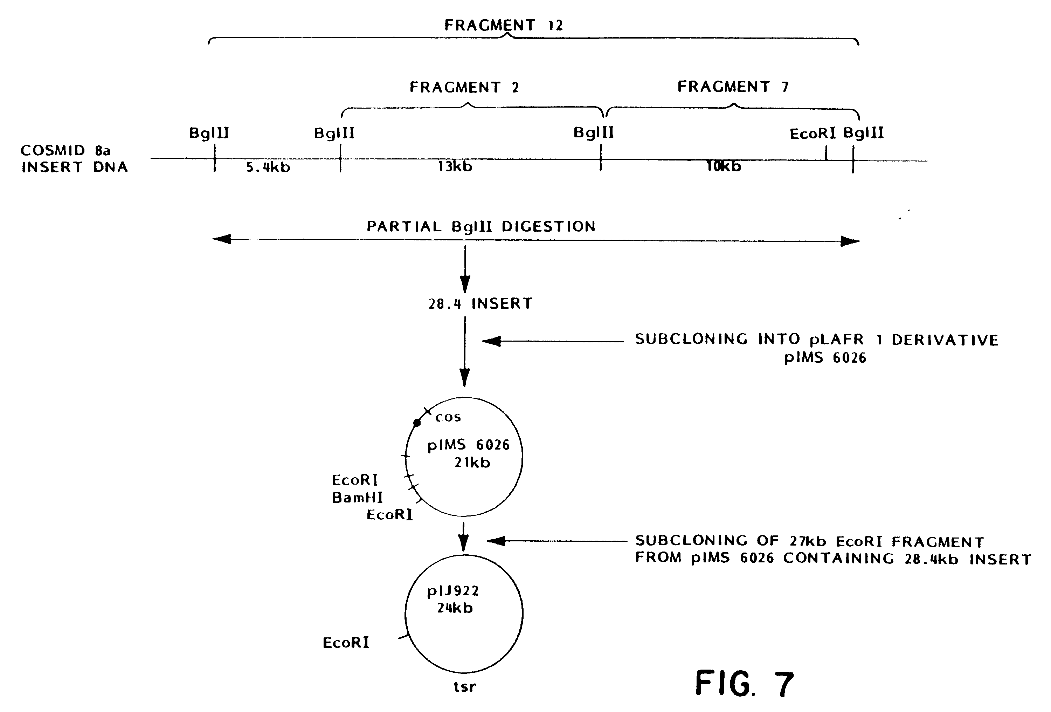

Almost the entire insert from clone 8a was subcloned as a 27 kb BglII fragment, fragment 12 in both orientations into plasmid pIJ922, as shown in Figure 7. Plasmid pIJ922 (Lydiate et al. (1985) Gene 35:223-235) is a low copy number Streptomyces vector capable of carrying large inserts. This vector was chosen in preference to the shuttle cosmid, used in clone bank preparation, to avoid deletion of the inserted sequences when inserting clone 8a directly into Streptomyces.

Fragment 8a was digested with BglII and a 13 kb BglII subfragment which contained the actinorhodin Gene III hybridizing sequence and a 10 kb BglII subfragment containing the actinorhodin Gene I hybridizing sequence were isolated. These subfragments were designated fragment 2 and fragment 7, respectively, as shown in figure 7. Digestion of the 8a fragment with BamHI allowed isolation of a 1,6 kb BamHI subfragment containing the Gene III hybridizing sequences, this subfragment was designated fragment 4 (Figure 7). Fragments 2, 7 and 4 were cloned into vector pIJ943, which is another low copy number Streptomyces vector (available from David Hopwood, john Innes Institute). Insertion of DNA fragments into the Bgl II cloning site of pIJ943 results in the loss of melanin production by the host bacterium, thus providing a convenient screen for insertion.

Two mutants of S. coelicolor which are blocked in actinorhodin Gene III (TK18) or Gene I (TK17) were obtained from David Hopwood, John Innes Institute. Each of these mutants was transformed with pIJ922, containing fragment 12 (27 kb), or pIJ943 containing fragment 2, fragment 7 or fragment 4. All manipulations of Streptomyces, including transformations, were performed essentially as described in Hopwood et al. (1985) Genetic Manipulation of Streptomyces - A Laboratory Manual, The John Innes Foundation. The results of these complementation experiments are shown in Table 1, Those fragments containing sequences that hybridize to actinorhodin Gene III were found to complement the S. coelicolor TK18 Gene III mutant. Fragment 10 which did not contain sequences that hybridize to Gene III did not complement the Gene III mutant. Similarly, fragment 7 which did not contain sequences that hybridized to the Gene I probe did not complement the Gene I mutant. Fragment 10 which contains sequences that hybridize to the Gene I probe appears to have complemented the TK17 Gene I mutant; however, the interpretation of the results of Table 1 is complicated since it has been found that the S. coelicolor TK17 Gene I mutant displays a "leaky" phenotype. Actinorhodin production was assayed using methods described in Malpartida and Hopwood, 1984, and Thompson et al. (1980) Nature 286:525-527.

Example 4: Introduction of Streptomyces sp. B41-146 DNA fragments into S. lividans

S. lividans contains the genes for production of actinorhodin, but does not normally produce this antibiotic, A strain of this species, S. lividans TK24 (Kieser et al. (1982) Mol. Gen. Genet. 185:223-228), was transformed with pIJ943 containing fragment 7 inserted in either orientation as well as with pIJ922 containing fragment 12 inserted in either orientation.

All S. lividans TK24 transformed with pIJ943 containg fragment 7 unexpectedly produced actinorhodin. Production of the antibiotic in transformants was found to be independent of the orientation of fragment 7. Fragment 7 therefore contains a DNA region that effects "switch-on" of silent actinorhodin genes in S. lividans. This result also strongly suggests that fragment 7 contains a DNA region that is involved in regulation of the production of milbemycin in Streptomyces sp. B41-146.

It was also found that introduction of fragment 12 into S. lividans, although only in one orientation effected production of actinorhodin.

A S. coelicolor mutant defective in actinorhodin Gene II, the putative regulatory gene for actinorhodin synthesis (Rudd and Hopwood (1979) J. Gen. Microbiol. 114:35-43; and Malpartida and Hopwood, 1986), was transformed with pIJ943 containing fragment 7. The resultant S. coelicolor transformants produced actinorhodin, indicating that milbemycin sequences in fragment 7 can also function in place of actinorhodin Gene II. By analogy to the actinorhodin genes, the milbemycin activating gene in fragment 7 is termed milbemycin Gene II.

It is interesting to note that even though the fragment 4 sequences functionally complement actinorhodin Gene II, no hybridization between fragment 7, milbemycin Gene II and actinorhodin Gene II sequences were detected. Sequences in fragment 7 are thus not structurally similar to those in Gene II.

Example 5: Extending the Streptomyces sp. B41-146 milbemycin gene cloned region to the right of fragment 8a

Since it appeared that fragment 7, which is at the far right end of 8a (Figure 6), contained a potential regulatory region for milbemycin production,it was possible that the milbemycin gene cluster extended to the right of sequences in fragment 8a. In order to clone Streptomyces sp. B41-146 regions to the right of fragment 8a, a 1.9 kb BamHI subfragment of 8a (fragment 14, Figure 6) was selected for use as a hybridization probe to a Streptomyces sp. B41-146 clone library, A second cosmid library was prepared with 20-30 kb DNA fragments from a partial Sau3a digest of B41-146 DNA and which were inserted into the cosmid vector pIMS6026, This second clone library was screened for hybridization to labelled fragment 14 probe cloned into the vector PGB2, obtained from Y. Nagamine. This vector was used because it showed no sequence homology to several commonly used E. coli plasmids, including pIMS6026 (Churchward, et al. (1984) Gene 31:165-171). In this case, Southern hybridization was carried out at 50°C with prehybridization for 3 hrs with 6xSSC/50% formamide/0.1% SDS/50µg/ml herring sperm DNA, and hybridization overnight using the same solution. Four sequential 1 hr washes were used. The first with 6xSSC/50% formamide; the second with 2xSSC/50% formamide; the third with 1xSSC/50% formamide; and the fourth with 0.5xSSC/50% formamide.

Four clones (out of 1,000 screened) were found to hybridize to the fragment 14 probe. Restriction enzyme analysis of these four clones, indicated that only two, pP0D1062 and pP0D1064, extended the sequence to the right of 8a (figure 8). The inserts of these clones were designated fragments 62 and 64, respectively, and positioning of these fragments with respect to fragment 8a and each other is shown in Figure 8.

Fragments 10, 8b, 8a, 62 and 64 define a Streptomyces sp. B41-146 region of about 62kb in length. It is believed that this DNA region contains the complete gene cluster for milbemycin production. A composite fragment MC (approximately 62 kb) which contains the contiguous DNA of the milbemycin gene cluster is constructed from fragments 10, 8b, 8a, 62 and 64 by appropriate restriction enzyme cleavage and ligation steps with intermediate fragments and the 62 kb MC fragment isolated by conventional DNA gel separation techniques.

Example 6: Insertional inactivation of Streptomyces sp. B41-146 milbemycin biosynthesis genes with cloned fragments

Confirmation that the cloned region (approximately 62 kb) contained genes for milbemycin production was obtained by insertional inactivation of the milbemycin genes of Streptomyces sp. B41-146 using fragments of the cloned region in homologous recombination experiments.

These experiments were based on the techniques employed by Chater and Bruton (1983) Gene 26:67-78. Basically, subfragments of the "milbemycin cluster" cloned region were inserted into an attachment site deleted defective phage vector. The recombinant phage vectors were then used to infect milbemycin producing Streptomyces sp. B41-146. Phage insertion into the chromosome was effected by homologous recombination events mediated by the homology of the inserted subfragment to the B41-146 chromosome. It is expected that only subfragments which are internal to a transcription unit will disrupt expression and result in a (milbemycin non-producing) mutant phenotype (Chater and Bruton, 1983). The resultant lysogens were cultured and subcultured several times on thiostrepton containing medium, and the surviving thiostrepton resistant bacteria were assayed for milbemycin production.

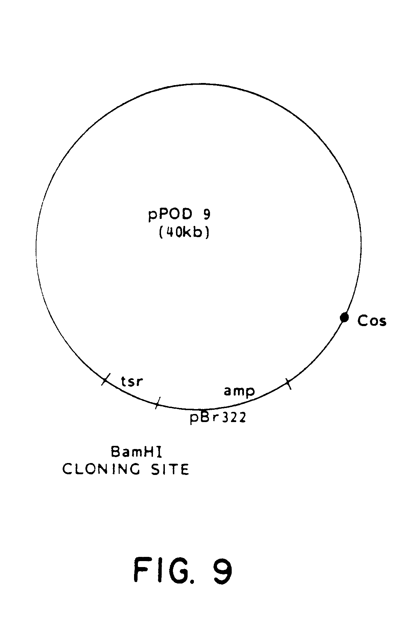

The phage vector used was phage pP0D9, the restriction map of which is shown if Figure 9. This vector is derived by insertion of the tsr (thiostrepton resistance) gene (Thompson et al. (1982) Gene 20:51-62) on a BclI-BamHI fragment into the BamHI site in the Tc gene of the $\phi$C31 derivative $\phi$C31$\pi$W17, in which the attachment site is deleted (Chater et al. (1981) Gene 15:249-256). pP0D9 contains the tsr gene adjacent to a unique BamHI site.

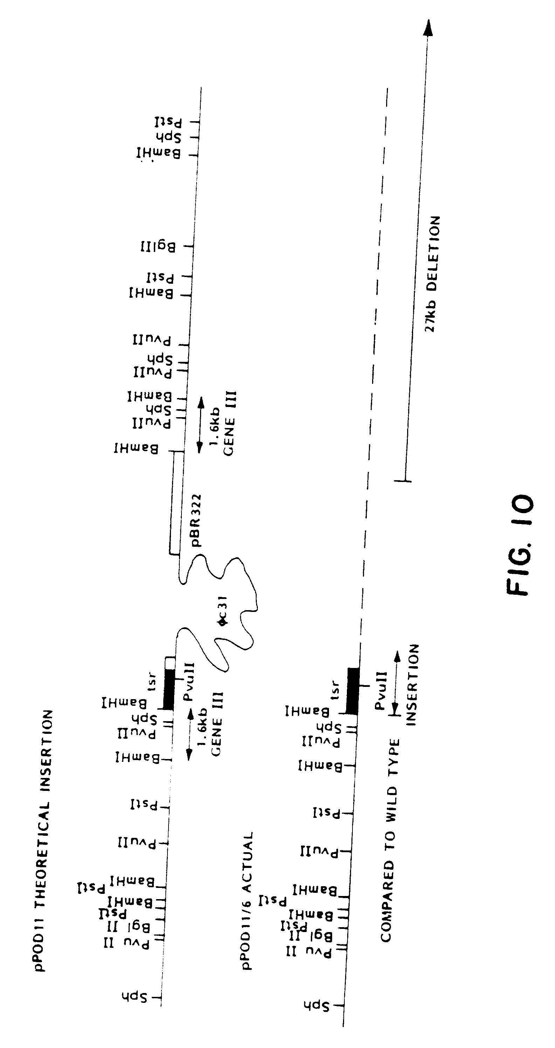

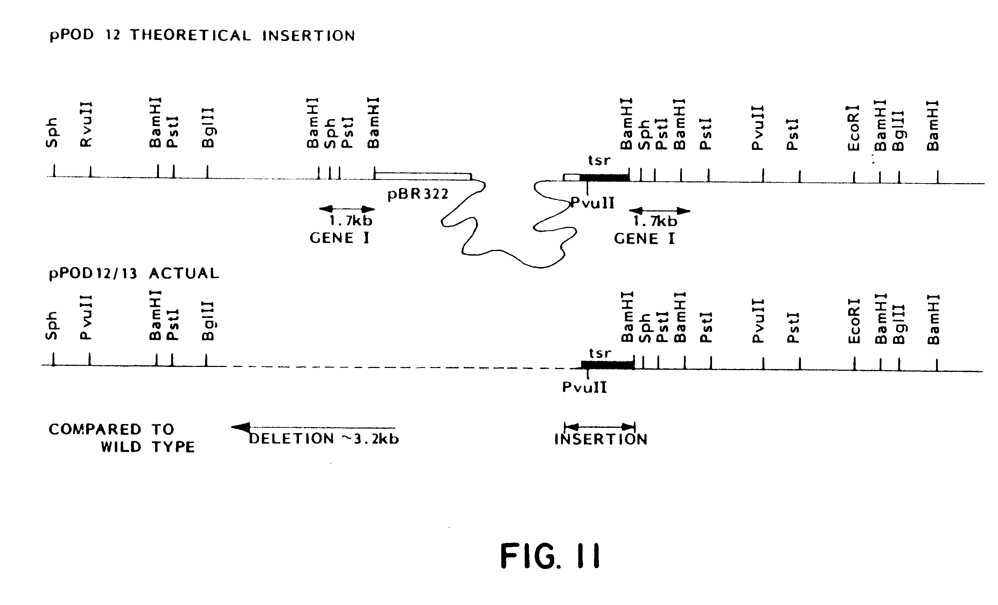

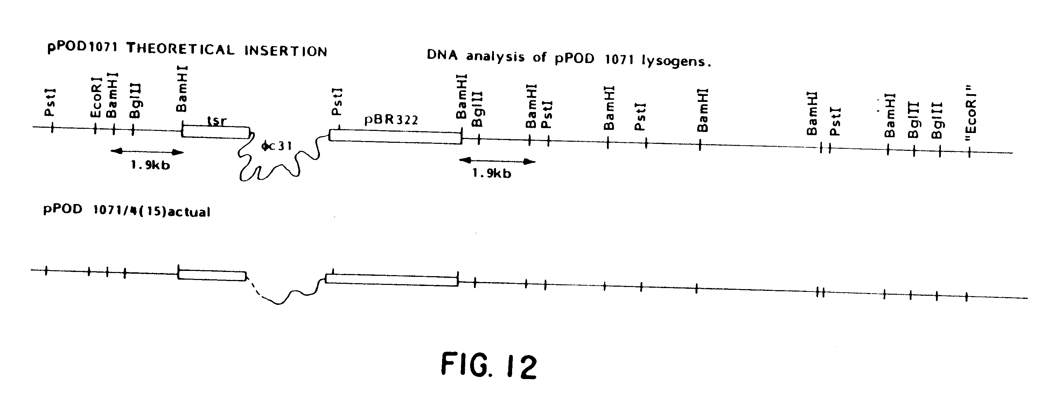

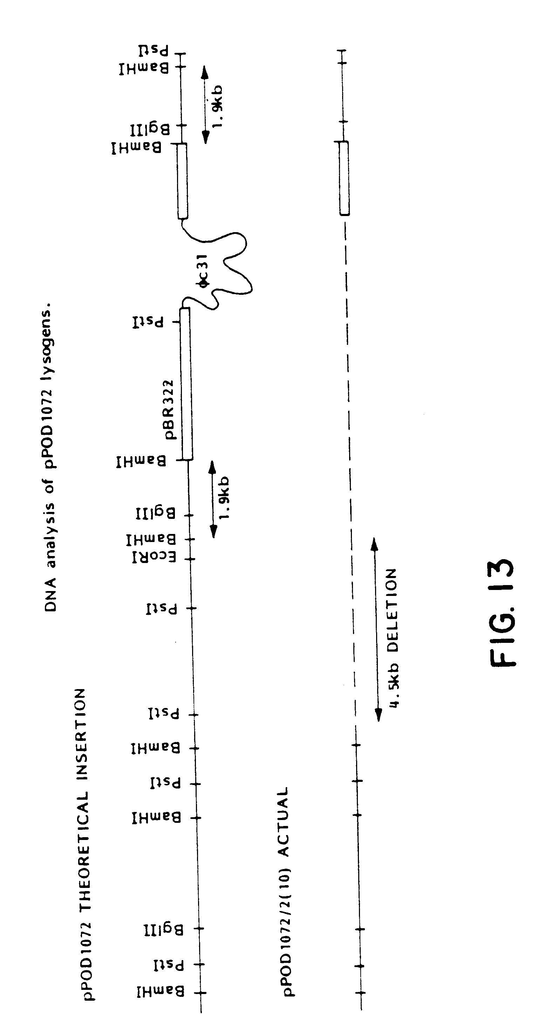

Fragment 4 (1.6 kb BamHI fragment) which contains milbemycin Gene III was cloned into pPOD9 in both orientations to give phages pPOD10 and pPOD11 (Figure 10). Fragment 16 (1.7 kb BamHI fragment) adjacent to Gene I was cloned to produce phage pPOD12 (Figure 11). Fragment 14 (1.9 kb BamHI fragment) was also cloned into pPOD9 in both orientations to give phages pPOD1071 and pPOD1072 (Figures 12 and 13). These phages were used to infect wild-type Streptomyces sp. B41-146, and in each case individual thiostrepton resistant lysogens were assayed for milbemycin production.

Contrary to expectations it was found that some lysogens resulting from infection with pPOD10 and pPOD11, which contain the entire milbemycin Gene III transcription unit, no longer produced milbemycin. Milbemycin production phenotype of individual lysogens was initially tested on plates assaying nematode killing (vide infra). Phenotypes were confirmed by analysis culture broth extracts for milbemycins (vide infra). In view of these unexpected results, DNA was prepared from several individual lysogens and examined by Southern blotting with several probes, in order to determine the structure of the DNA that had been inserted. It was found, as exemplified for lysogen pPOD11/6 (which resulted from "insertion" of pPOD11), that although the tsr gene had been incorporated from the phage, the milbemycin gene cluster had been disrupted by deletion of not only part of the phage DNA but also the milbemycin Gene III duplicate copy as well as chromosomal DNA further to the right of the site of recombination including sequence with the milbemycin Gene I region. The deletion which occurred in pPOD11/6 (non-producing lysogen) compared to the expected insertion are shown in Figure 10.

Similarly, infection of Streptomyces sp. B41-146 with pPOD12 resulted in lysogens defective for milbemycin production. Again, deletions of phage and chromosomal DNA were observed on DNA analysis of selected lysogens. For example, Figure 11 compares the deletion in pPOD12/13 to the expected phage insertion.

After infection of Streptomyces sp. B41-146 with pPOD1071, onty about 10% of the resultant lysogens were defective for milbemycin production, while infection with pPOD1072 resulted in a lysogen population with 68% loss of milbemycin production. Figure 12 compares the expected insertion of pPOD1071 to the structure of lysogen pPOD1071/4(15). This lysogen, which suffered only a deletion of $\phi$31 sequences, retains production of milbemycin. Figure 13 compares the expected insertion of pPOD1072 with the structure of lysogen pPOD1072/2(10)which does not produce milbemycin. Again, a deletion of phage and chromosomal DNA was found in this lysogen.

Thus, the infection experiments with phage DNA containing fragments from the milbemycin gene clones has shown that these fragments do contain genes essential for milbemycin production, since deletions in the chromosomal region at or near the location of these genes result in loss of milbemycin production.

The phage infection technique can therefore be used to create a variety of milbemycin non-producing mutants by DNA deletions throughout the milbemycin biosynthetic cluster region. These mutants can be used in order to further investigate the genetics of milbemycin production and delineate other specific milbemycin genes within the milbemycin cluster region.

Example 7: Assays for milbemycin production

A plate assay for milbemycin production by bacteria was developed based on the nematicidal activity of milbemycin, and is herein exemplified for milbemycin production by Streptomyces sp. B41-146.

Streptomyces sp. B41-146 are plated on C agar medium (Goegelman et al . (1982) EPO Patent 058 518) at a density which allows formation of separate single colonies. A suspension of nematodes (Caenorhabditis elegans) is added to the test plate containing bacterial colonies. Plates are initially examined for nematode density and viability and again after 3-10 hours for nematode killing.

C. elegans is cultured as described in Brenner (1974) Genetics 77:71-94. NG agar is seeded with the E. coli uracil auxotroph 0P50 in order to produce a bacterial lawn after incubation at 37°C for 18 hours.

A single hermaphrodite nematode (C. elegans strain N2) is added to the bacterial lawn and the plate is incubated for 5 days at room temperature. After incubation the plate contains a dense population of C. elegans, which is then harvested using distilled water.

Milbemycin production was also assayed using an HPLC method described in Takiguchi et al. (1983) J. Antibiotics 36:502-508.

Seed medium 50 ml) in a 250 ml baffled flask was inoculated with 50µl of spores of the strain to be examined. The culture was incubated with shaking at 28°C for 12 days (Takiguchi et al., 1983).

After incubation the total culture was extracted with acetone. Acetone extracts were concentrated 10-fold and 100µl aliquots of the concentrated extract were applied to a C18 reverse-phase HPLC column for analysis. HPLC conditions used are those described in Takiguchi et al., 1983. Column effluent was monitored at 240 nm.

Those skilled in the art will appreciate that the invention described herein and the methods described are susceptible to variations and modification other than as specifically described. It is to be understood that the invention includes all such variations and modifications which fall within its spirit and scope.

Table I: Complementation of Act⁻ mutants with fragments cloned from Streptomyces sp. B41-146

Streptomyces sp. B41-146 Fragments

| Act mutant | 12 (27kb) | 2 (13kb) | 7 (10kb) | 4 (1.6kb) |
|---|---|---|---|---|
| TK17 | not tested | - | (+) | not tested |
| TK18 | + | + | - | + |

+ = complementation        - = no complementation

(+) = complementation variable; TK17 mutation is leaky

## Claims

1. A method for producing a milbemycin in a naturally non-milbemycin producing strain of a bacterium of the genus Streptomyces which comprises introducing into said non-producing strain a DNA fragment which comprises the DNA composite fragment MC, as depicted in Figure 8, or a DNA fragment that is functionally equivalent thereto.

2. A method according to claim 1 wherein said naturally non-milbemycin producing strain is a strain of Streptomyces lividans.

3. A method for activating expression of a polyketide antibiotic biosynthetic gene cluster in a bacterial strain of the genus Streptomyces which strain contains the biosynthetic genes sufficient for production of said polyketide antibiotic but lacks a functional polyketide antibiotic biosynthesis activator gene which comprises introducing into said bacterial strain a DNA fragment which comprises milbemycin Gene II, as described with reference to Figure 8.

4. A method according to claim 3 wherein said bacterial strain is a strain of Streptomyces lividans.

5. A method according to claim 4 wherein said polyketide antibiotic is actinorhodin.

6. A method according to claim 3 wherein said bacterial strain is a strain of Streptomyces coelicolor and said polyketide antibiotic is actinorhodin.

7. A method according to claim 3 wherein said bacterial strain is a strain of Streptomyces avermitilis and said polyketide antibiotic is an avermectin.

8. A vector which comprises a DNA fragment consisting essentially of the milbemycin biosynthetic gene cluster.

9. A vector according to claim 8 wherein said DNA fragment is the fragment MC, as depicted in Figure 8, or a DNA fragment that is functionally equivalent thereto.

10. A vector which comprises a DNA molecule consisting essentially of the DNA sequence encoding a milbemycin gene selected from the group consisting of milbemycin Gene I, milbemycin Gene II, and milbemycin Gene III, as described with reference to Figure 8.

11. A vector according to claim 8 wherein said DNA fragment is a DNA fragment selected from the group of DNA fragments consisting of fragment 1, fragment 2, fragment 3, fragment 4, fragment 5, fragment 6, fragment 7, fragment 8a, fragment 8b, fragment 10, fragment 12, fragment 14, fragment 16, fragment 62 and fragment 64, as depicted in Figures 6 to 8.

12. A bacterial strain containing the vector of any of claims 8 to 11.

13. A hybridization probe molecule consisting essentially of the milbemycin biosynthetic gene cluster.

14. A hybridization probe molecule that is fragment MC, as depicted in Figure 8, or a DNA fragment that is functionally equivalent thereto.

15. A hybridization probe consisting essentially of the DNA sequence encoding a milbemycin gene selected from the group of milbemycin genes consisting of milbemycin Gene I, milbemycin Gene II, and milbemycin Gene III, as depicted in Figure 8.

16. A hybridization probe that is a DNA fragment selected from the group of DNA fragments consisting of fragment 1, fragment 2, fragment 3, fragment 4, fragment 5, fragment 6, fragment 7, fragment 8a, fragment 8b, fragment 10, fragment 12, fragment 14, fragment 16, fragment 62 and fragment 64, as depicted in Figures 6 to 8.

17. A hybridization probe, the sequence of which is derived from the sequence of a fragment selected from the group of fragments consisting of fragment 1, fragment 2, fragment 3, fragment 4, fragment 5, fragment 6, fragment 7, fragment 8a, fragment 8b, fragment 10, fragment 12, fragment 14, fragment 16, fragment 62 and fragment 64, as depicted in Figures 6 to 8.

18. A DNA fragment selected from the group of DNA fragments consisting of fragment 1, fragment 2, fragment 3, fragment 4, fragment 5, fragment 6, fragment 7, fragment 8a, fragment 8b, fragment 10, fragment 12, fragment 14, fragment 16, fragment 62 and fragment 64, as depicted in Figures 6 to 8.

POLYKETIDE PATHWAY

FIG. 1

ACTINORHODIN

NYSTATIN

AVERMECTIN A₁ₐ

MILBEMYCIN α₁(A₃)

FIG. 2

FIG. 3

EP 0 463 707 A1

FIG. 4

1. <u>S. coelicolor</u>

2. <u>S. lividans</u>

3. <u>Streptomyces</u> sp. B41–146 (Milbemycin producer)

FIG. 5

vph   =   viomycin phosphotransferase
amp  =    ampicillin
tsr    =   thiostrepton
mel   =   melanin

FIG. 6

FIG. 7

FIG. 8

pPOD 9
(40kb)

Cos

tsr

amp

pBr322

BamHI
CLONING SITE

FIG. 9

FIG. 10

pPOD 12 THEORETICAL INSERTION

Sph
PvuII
BamHI
PstI
BglII
BamHI
Sph
PstI
BamHI
tsr
BamHI
Sph
PstI
BamHI
PstI
PvuII
PstI
EcoRI
BamHI
BglII
BamHI

pBR322
PvuII

1.7kb
GENE I

PvuII

1.7kb
GENE I

pPOD 12/13 ACTUAL

Sph
PvuII
BamHI
PstI
Bglī
BamHI
Sph
PstI
BamHI
tsr
PstI
PvuII
PstI
EcoRI
BamHI
BglII
BamHI

PvuII

COMPARED TO
WILD TYPE

DELETION ~3.2kb

INSERTION

FIG. 11

EP 0 463 707 A1

FIG. 12

DNA analysis of pPOD 1072 lysogens.

pPOD1072 THEORETICAL INSERTION

pPOD1072/2(10) ACTUAL

4.5kb DELETION

FIG. 13

EP 0 463 707 A1

European Patent Office

# EUROPEAN SEARCH REPORT

Application Number

EP    91 20 2030

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4 ) |
|---|---|---|---|
| Y | EP-A-118 367 (MERCK)<br>* page 10, line 28 - page 11, line 14; claims; example 2 * | 1 | C12N15/52<br>C12N15/76<br>C12P17/02<br>C12Q1/68<br>C12N1/20 |
| D,Y | GENE.<br>vol. 26, 1983, AMSTERDAM NL<br>pages 67 - 78;<br>K.F.CHATER ET AL.: 'Mutational cloning in Streptomyces and the isolation of antibiotic production genes '<br>*page 76*<br>* page 72, line 8 - page 74, line 12 * | 1 | |
| D,A | NATURE.<br>vol. 309, no. 5967, May 31, 1984, LONDON GB<br>pages 462 - 464;<br>F.MALPARTIDA ET AL.: 'Molecular cloning of the whole biosynthetic pathway of a Streptomyces antibiotic and its expression in a heterologous host '<br>* the whole document * | 1 | |
| D,P, Y | BIOTECHNOLOGY<br>vol. 4, no. 3, March 1986, NEW YORK US<br>pages 229 - 232;<br>R.STANZAK ET AL.: 'Cloning and expression in Streptomyces lividans of clustered erythromycin biosynthesis genes from Streptomyces erythreus '<br>* the whole document * | 1 | TECHNICAL FIELDS SEARCHED (Int. Cl.4 )<br><br>C12N<br>C12P<br>C12Q |
| P,A | BIOTECHNOLOGY<br>vol. 4, no. 9, September 1986, NEW YORK US<br>pages 786 - 789;<br>J.T. FAYERMAN: 'New developments in gene cloning in antibiotic producing microorganisms '<br>* the whole document * | 1 | |

The present search report has been drawn up for all claims

| Place of search | Date of completion of the search | Examiner |
|---|---|---|
| THE HAGUE | 07 OCTOBER 1991 | DELANGHE L. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)